Europäisches Patentamt

European Patent Office

Office européen des brevets

(19)

(11) Veröffentlichungsnummer: **0 417 619 A1**

## (12) EUROPÄISCHE PATENTANMELDUNG

(21) Anmeldenummer: 90117037.3

(22) Anmeldetag: 05.09.90

(51) Int. Cl.⁵: $C11D$ $1/32$

(30) Priorität: 07.09.89 DE 3929740

(43) Veröffentlichungstag der Anmeldung:
20.03.91 Patentblatt 91/12

(84) Benannte Vertragsstaaten:
BE CH DE ES FR GB IT LI NL SE

(71) Anmelder: HOECHST AKTIENGESELLSCHAFT
Postfach 80 03 20
W-6230 Frankfurt am Main 80(DE)

Anmelder: Deutsche Gelatine-Fabriken
Stoess & Co.GmbH
Postfach 100 Gammelsbacher Strasse 2
W-6930 Eberbach(DE)

(72) Erfinder: Turowski, Angelika, Dr.
Im Wiesenthal 4
W-6930 Eberbach(DE)
Erfinder: Quack, Jochen Meinhard, Dr.
Wilhelm-Reuter-Strasse 16
W-6290 Eppstein/Taunus(DE)
Erfinder: Reng, Alwin
Im Schulzehnten 22
W-6233 Kelkheim (Taunus)(DE)
Erfinder: Holst, Arno, Dr.
Drususstrasse 3
W-6200 Wiesbaden(DE)

(54) Hochmolekulare Eiweissfettsäurekondensationsprodukte mit sehr guter Haut- und Schleimhautverträglichkeit.

(57) Hochmolekulare Eiweißfettsäurekondensationsprodukte erhalten durch Umsetzung eines Mols eines Eiweißhydrolysats mit einer mittleren Molmasse von 3000 bis 7000 mit 0,5 bis 3, vorzugsweise 2 bis 2,5 Mol eines $C_{12}$-$C_{18}$-Fettsäurechlorids in wäßrigem Medium bei einem pH-Wert von 7 bis 12. Diese Eiweißfettsäurekondensationsprodukte zeichnen sich dadurch aus, daß sie keinerlei Reizung der Schleimhäute hervorrufen. Sie eignen sich deshalb hervorragend als Tenside für milde Wasch- und Reinigungsmittel.

EP 0 417 619 A1

EP 0 417 619 A1

## HOCHMOLEKULARE EIWEISSFETTSÄUREKONDENSATIONSPRODUKTE MIT SEHR GUTER HAUT- UND SCHLEIMHAUTVERTRÄGLICHKEIT

Seit einigen Jahren beobachtet man weltweit den Trend, bei der Konzipierung von kosmetischen Wasch- und Reinigungsmitteln wie Shampoos, Badezusätze, Waschlotionen und Babypflegeprodukte dermatologisch einwandfreie Pflegepräparate zu entwickeln, indem die Haut- und Schleimhautverträglichkeit der Grundtenside durch Kombination mit anderen milden Tensiden und/oder durch Additive verbessert wird.

Im allgemeinen handelt es sich bei der Reinigung der menschlichen Haut, der Haare und der Zähne um anionische Tenside, wie z.B. Seifen, Alkylsulfate, Alkansulfonate, Fettsäureisethionate, $\alpha$-Olefinsulfonate, Methyltauride, Sarkoside, Acylglutamate und/oder nichtionische Tenside wie beispielsweise Fettalkoholpolyglykolether und/oder amphotere Tenside wie Alkylbetaine, Alkylamidobetaine, Imidazolin-Abkömmlinge und kationische Tenside wie beispielsweise Alkyltrimethylpentaoxethylammoniumlaktat, die allein oder in Kombination untereinander angewandt werden.

Einige dieser Tenside insbesondere Alkylsulfate, $\alpha$-Olefinsulfonate, Alkylbetaine, Alkylamidobetaine werden wegen ihrer sehr guten Schaum- und Reinigungswirkung und ihrer günstigen Preise in hohem Maße eingesetzt. Ihr Nachteil liegt aber in ihrer relativ schlechten Haut- und Schleimhautverträglichkeit. Denn ein besonders wichtiges Kriterium für die Verwendung der Tenside zur Herstellung von milden Haarshampoos, Duschbädern, Intimwaschmitteln, klassischen Seifen und sogenannten Syndetseifen sowie Babyreinigungspräparaten ist das Vorhandensein einer sehr guten Schleimhautverträglichkeit.

Bekannt ist auch die Verwendung von Eiweißfettsäurekondensaten mit niedriger bis mittlerer Molmasse, beispielsweise bis Molmasse 700. Diese Eiweißfettsäurekondensate können in bekannter Weise durch Acylierung von Eiweißhydrolysaten mit Fettsäurechloriden nach Schotten-Baumann oder durch Kondensation der entsprechenden Fettsäuremethylester mit Eiweißhydrolysaten synthetisiert werden. In der Literatur wird darauf hingewiesen, daß diese Eiweißfettsäurekondensate mit niedriger bis mittlerer Molmasse von etwa 500-700 eine gute Waschwirkung besitzen, die mit steigender Molmasse stark abnehmen soll. Dagegen soll die Reizung der Schleimhäute mit steigender Molmasse nur noch wenig abnehmen.

Überraschenderweise wurde jedoch gefunden, daß Eiweißfettsäurekondensate mit einer höheren Molmasse von beispielsweise 3000-7000 eine weitaus bessere Haut- und Schleimhautverträglichkeit als die entsprechenden Eiweißfettsäurekondensate mit niedriger bis mittlerer Molmasse besitzen. Auch die Wasch- und Reinigungswirkung ist im Vergleich zu den niedrigmolekularen Eiweißfettsäurekondensaten nur unwesentlich geringer und für die praktische Anwendung voll ausreichend.

Gegenstand der Erfindung sind hochmolekulare Eiweißfettsäurekondensationsprodukte, erhalten durch Umsetzung eines Mols eines Eiweißhydrolysats mit einer mittleren Molmasse von 3000-7000 mit 0,5 - 3,0, vorzugsweise 2,0 - 2,5 Mol eines $C_{12}$-$C_{18}$-Fettsäurechlorids in wäßrigem Medium bei einem pH-Wert von 7 bis 12 sowie die Verwendung dieser Produkte in Wasch- und Reinigungsmitteln und Wasch- und Reinigungsmittel, welche diese Produkte enthalten.

Die zugrundeliegenden Eiweißhydrolysate werden auf üblichem Weg erhalten durch Hydrolyse von höhermolekularen tierischen und pflanzlichen Proteinen, z.B. Kollagenprotein aus Bindegewebe, Fleischhydrolysat, Blut, Milchproteine und Sojaprotein. Besonders günstig sind Eiweißhydrolysate, die aus den Bindegeweben von Säugetieren wie Tierhäute, aus Knochen, aus Leime, sowie aus in Gerbereien als Nebenprodukte anfallenden Chromfalzspänen und Maschinenleimleder gewonnen werden. Diese Produkte werden durch einen chemischen oder vorzugsweise durch einen enzymatischen Prozeß zu Eiweißhydrolysaten der oben angegebenen Molmasse hydrolysiert. Die angegebenen Werte für die rechnerisch ermittelten mittleren Molmassen wurden aus der Molekulargewichtsverteilung mittels Hochdruckflüssigkeitschromatographie (HPLC) unter Zuhilfenahme von kollagenen Eichpeptiden als Standard erhalten.

Diese Eiweißhydrolysate werden nach üblichen Verfahren (Schotten-Baumann) mit $C_{12}$-$C_{18}$-Fettsäurechloriden in wäßriger Lösung bei einem pH-Wert von 7-12, vorzugsweise 8-9,5, acyliert. Man erhält wäßrige Lösungen mit einem Feststoffgehalt von bis zu 60 Gew.-%, vorzugsweise mit einem Feststoffgehalt von 30 bis 40 Gew.-%. Diese wäßrigen Lösungen lassen sich direkt weiterverarbeiten, man kann die Eiweißfettsäurekondensationsprodukte aber auch ohne Probleme aus den wäßrigen Lösungen als Pulver isolieren, beispielsweise durch Sprühtrocknung.

Die so erhaltenen Eiweißfettsäurekondensationsprodukte zeigen überraschenderweise keinerlei Reizung der Haut, Augen und Schleimhäute, wie es bei Eiweißfettsäurekondensationsprodukten der Fall ist, die auf Eiweißhydrolysaten mit einer Molmasse von 200-500 aufbauen.

Die erfindungsgemäßen Produkte sind nicht toxisch, nicht sensibilisierend, nicht irritierend, üben eine reizhemmende Wirkung in Kombination mit gängigen Tensiden aus, zeigen gute Kompatibilität mit anionischen und nichtionischen Tensiden sowie teilweise auch mit quartären Ammoniumverbindungen. Sie weisen

2

selbst eine milde Reinigungswirkung auf und eignen sich daher sehr gut als Tensid in Wasch- und Reinigungsmitteln, wie z.B. in Shampoos, Duschbädern, Schaumbädern, Syndetseifen, flüssigen Seifen, Haushaltsreinigern, Spülmitteln, Geschirrspülmitteln, Waschmitteln, Schutzpastenseifen.

Gegenstand der Erfindung ist somit auch ein Wasch- und Reinigungsmittel, welches das Eiweißkondensationsprodukt enthält.

## Beispiel 1

600 g einer 30 %igen wäßrigen Kollagenhydrolysat-Lösung mit einer mittleren Molmasse von 3000 wird mit Natronlauge auf einen pH-Wert von 9,5 eingestellt.

In diese Kollagenhydrolysat-Lösung werden anschließend 63 g 98 %iges Cocoylchlorid eingebracht. Nach Beendigung der Säurechloridzugabe wird die Lösung auf 50°C erwärmt und 120 Min. bei dieser Temperatur gehalten.

Anschließend wird das Reaktionsgemisch mit destilliertem Wasser auf 35 % Trockensubstanzgehalt eingestellt und mit Salzsäure der pH-Wert auf 6,5 gebracht. Nach Abkühlung auf Raumtemperatur kann der Zusatz der Konservierungsmittel und Substanzen zur Geruchsverbesserung und zur Verbesserung der Transparenz erfolgen. Das so erhaltene Produkt wird im folgenden mit EFK 3000 bezeichnet.

Die entsprechend dem Beispiel 1 hergestellten hochmolekularen Eiweißfettsäurekondensate besitzen, wie die nachstehenden Darstellungen zeigen, eine Reihe von anwendungstechnischen Eigenschaften, die die Herstellung von kosmetischen, pharmazeutischen und technischen Reinigungsmitteln mit sehr guter Haut- und Schleimhautverträglichkeit ermöglichen.

## Beispiel 2

1400 g einer 50 %igen wäßrigen Kollagenhydrolysat-Lösung mit einer mittleren Molmasse von 7000 wird mit Natronlauge auf einen pH-Wert von 11 eingestellt. In diese Lösung läßt man 47,4 g 98 %iges Cocoylchlorid unter intensivem Rühren eintropfen. Nach Beendigung der Säurechloridzugabe wird die Lösung auf 60°C erwärmt und 120 Min. bei dieser Temperatur gehalten.

Anschließend wird der pH-Wert der Lösung mit Salzsäure auf 6,2 eingestellt und der Trockensubstanzgehalt mit Wasser auf 30 %.

Nach Abkühlung auf Raumtemperatur wird das Produkt konserviert.

Oberflächenspannung

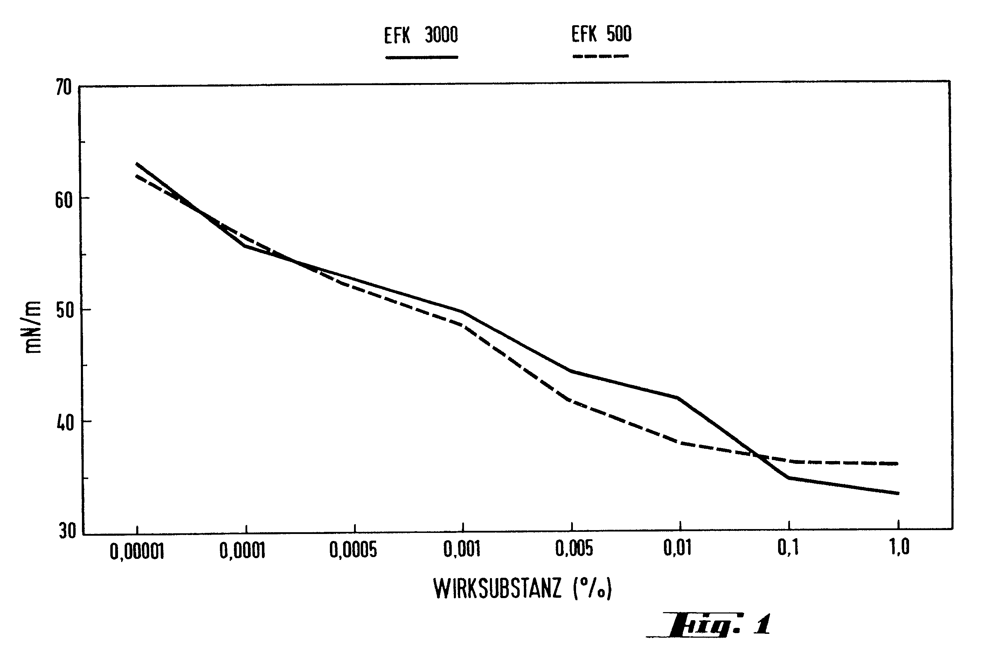

Die Bestimmung der Oberflächenspannung erfolgte nach der Ringabreißmethode mit einem Digital-Tensiometer (Krüss, K 10 ST) bei 20°C in destilliertem Wasser. Für die Bestimmung wurde die nach Beispiel 1 hergestellte Substanz eingesetzt. Die Werte sind aus der Figur 1 zu ersehen, wobei die erfindungsgemäße Substanz mit EFK 3000 bezeichnet wurde. Bei der Vergleichssubstanz, mit EFK 500 bezeichnet, handelt es sich um ein Eiweißfettsäurekondensat entsprechend dem Stand der Technik mit einer mittleren Molmasse von 500. Es ist zu erkennen, daß auch das höher molekulare EFK 3000 die in der Praxis gewünschte Verringerung der Oberflächenspannung aufweist.

Schaumverhalten nach Ross-Miles

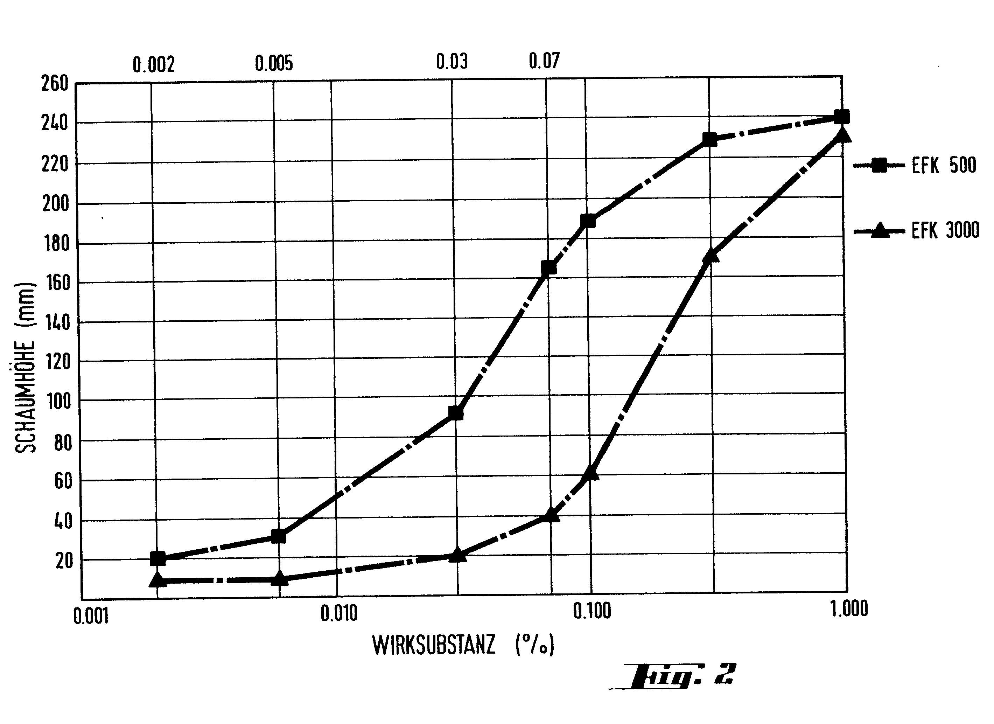

In Anlehnung an die Methode von Ross-Miles wurde das Schaumverhalten von EFK 3000 entsprechend dem Beispiel 1 geprüft. Die Schaumkurve ist in Figur 2 dargestellt. In den praxisüblichen Konzentrationen zwischen 0,5 und 1 % ist ein gutes Schaumverhalten vorhanden; besonders hervorzuheben ist die im allgemeinen gewünschte Feinblasigkeit des Schaumes und die damit verbundene Zeitstabilität.

Stabilität

Für den praktischen Einsatz wird eine Stabilität der Tenside gegenüber UV-Licht, höheren Temperaturen, z.B. +40°C und eine pH-Beständigkeit verlangt. Wie die nachstehende Tabelle 1 zeigt, besitzt EFK 3000 entsprechend dem Beispiel 1 sowohl eine gute Farbbeständigkeit als auch eine sehr gute pH-Stabilität nach 6-monatiger Lagerung bei Raumtemperatur und $+40\,^\circ$ C.

Tabelle 1

|  | Stabilitätsprüfung EFK 3000 | | |
|---|---|---|---|
| Farbbeständigkeit | | | |
|  | Dunkel | $40\,^\circ$ C | Licht |
| Jodfarbzahl bei Testbeginn | 8 | 8 | 8 |
| Jodfarbzahl nach 2 Monaten | 8 | 8 | 8 |
| Jodfarbzahl nach 6 Monaten | 8 | 9 | 8 |
| pH-Stabilität | | | |
|  | $+20\,^\circ$ C | $+40\,^\circ$ C |  |
| pH (10 %ig) bei Testbeginn | 7,44 | 7,44 | |
| pH (10 %ig) nach 2 Monaten | 7,28 | 7,26 | |
| pH (10 %ig) nach 6 Monaten | 7,28 | 7,26 | |

Waschvermögen

Das Waschvermögen wurde einmal an angeschmutztem Baumwollgewebe und zum anderen an der menschlichen Haut getestet.

Pigment-Waschvermögen

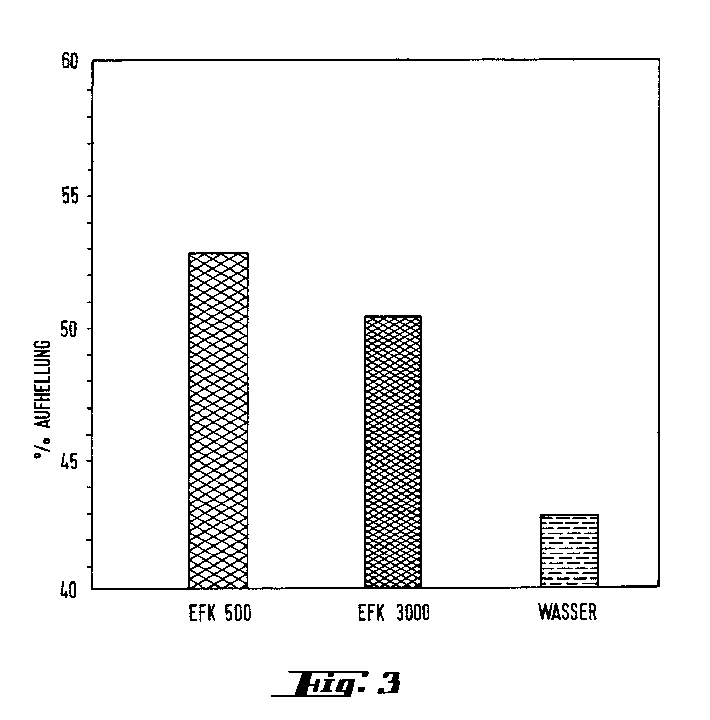

Mit dem Launder-Ometer wurde unter folgenden Bedingungen die Waschwirkung am Gewebe bestimmt. Baumwollgewebe angeschmutzt mit Hautfett/Pigment.
Waschtemperatur $30\,^\circ$ C,
Waschzeit 5 Min.
Konzentration 10 g/l,
Wasserhärte $15\,^\circ$ dH.,
pH-Wert 6,5.
Wie die Figur 3 veranschaulicht, besitzt das EFK 3000 noch eine für den Einsatz in Reinigungsmitteln ausreichende Waschwirkung, die nur geringfügig unter dem Wert des niedrig molekularen EFK 500 liegt.

Reinigungseffekt an der menschlichen Haut

Die Bestimmung des Wascheffektes mit der Hautwaschmaschine nach Tronnier arbeitet mit einem ganz bestimmten Modellschmutz, der fett- und wasserlösliche Stoffe sowie Pigmente enthält, um die Gesamtheit der Tensidwirkung zu erfassen.

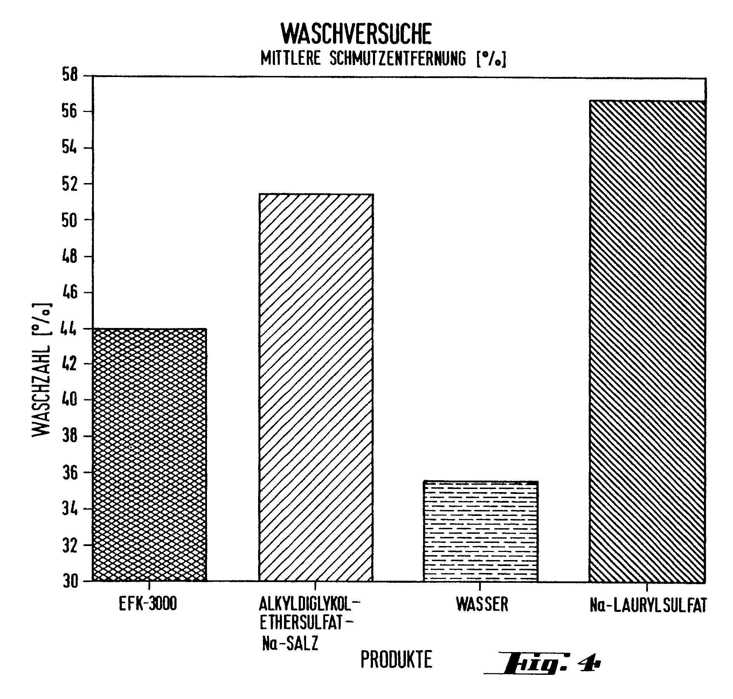

Die Figur 4 veranschaulicht das befriedigende Waschvermögen von EFK 3000 im Vergleich zu den Tensiden Na-Laurylsulfat und Alkyldiglykolethansulfat-Na-Salz sowie reinem Wasser (Waschzahl ist definiert als Wascheffekt minus Beschmutzungseffekt).

Biologischer Abbau

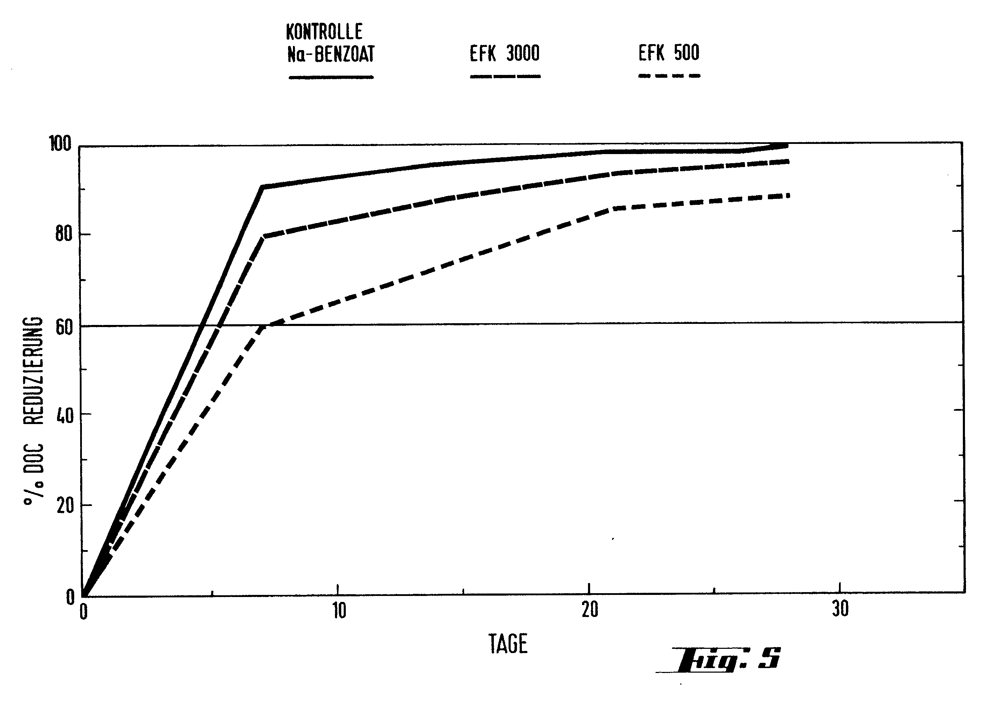

Die Bestimmung des biologischen Abbaus erfolgte nach dem modifizierten OECD-Screening Test 301 E, bei dem analytisch die biologische Abbaurate der zu prüfenden Substanz verfolgt wird (DOC-Reduzierung = Dissolved Organic Carbon). Die Abbaukurven der Figur 5 zeigen, daß das EFK 3000 wesentlich schneller abbaubar ist, als das entsprechende niedrig molekulare EFK 500. Schon nach 5 Tagen Prüfzeit erreicht EFK 3000 den vom deutschen Gesetzgeber zukünftig festgesetzten Wert von 60 % Totalabbau = Mineralisierung zu Kohlendioxid und Wasser.

Zein-Test

Der Zein-Test (Protein des Maiskorns) liefert zuverlässige Informationen über die entfettenden und hautreizenden Eigenschaften von Tensiden. Zwischen der Hautverträglichkeit von Tensiden und der Solubilisierung von Zein besteht eine Korrelation, danach sind Tenside mit hohen Zeinwerten stärker entfettend und hautreizend und üben somit einen stärkeren Einfluß auf die Hautrauhigkeit aus. Auch der Zusammenhang mit der kritischen Micellkonzentration der Tenside wird in der Literatur beschrieben. Tenside mit hohen CMC-Werten weisen auch hohe Zeinwerte auf und zeigen im Duhring-Kammer-Test auch die höchste Reizwirkung.

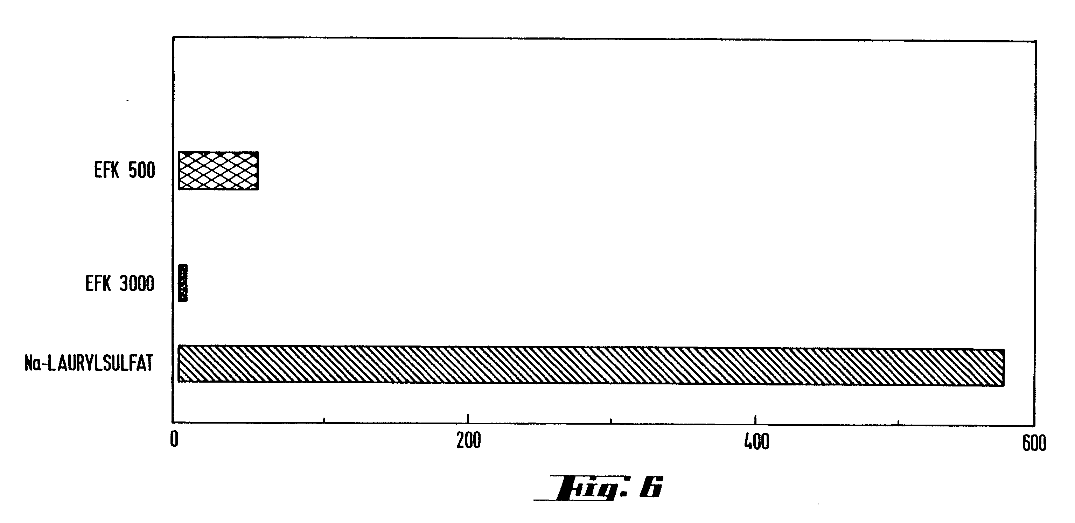

Dieser in vitro-Test zeigt beispielsweise entsprechend der Figur 6, daß das allgemein als aggressiv geltende Natriumlaurylsulfat einen sehr hohen Wert ergibt, während EFK 3000, auch im Vergleich zu EFK 500, keine Zeinlöslichkeit aufweist.

Albumindenaturierungstest

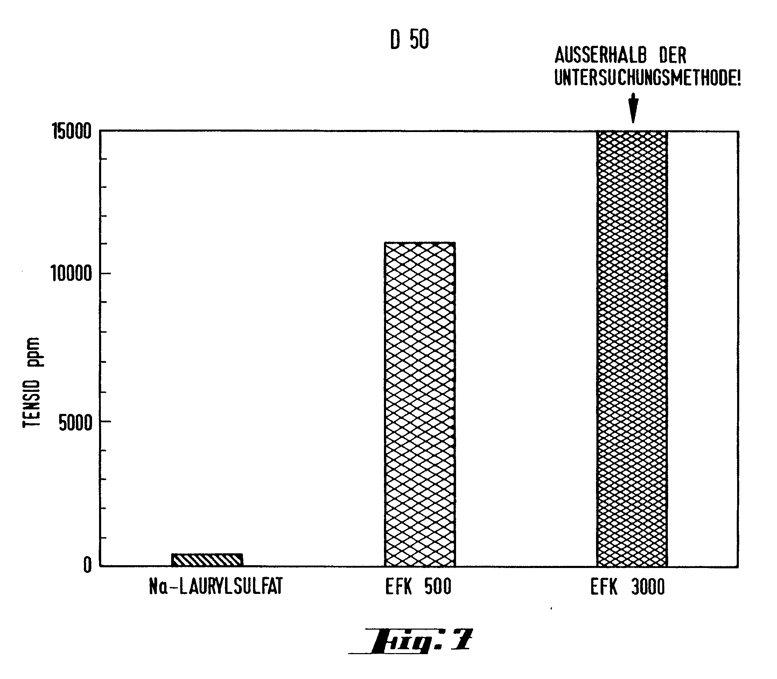

Der Albumindenaturierungstest ist ebenfalls ein Maßstab zur Charakterisierung der Hautverträglichkeit von Tensiden. Die Ergebnisse dieses Tests zeigen eine gute Korrelation zu den Ergebnissen von Hautrauhigkeitsmessungen (Methylenblaumethode, Bildanalyse). Je höher der $D_{50}$-Wert ist, umso geringer ist die eiweißschädigende Wirkung des Produkts und umso günstiger sind nach Frosch die dermatologischen Eigenschaften in vivo. Die Figur 7 zeigt die große Überlegenheit des hochmolekularen Eiweißtensids EFK 3000 zum Vergleichsprodukt EFK 500. Das EFK 3000 sprengt den Rahmen der Untersuchungsmethode, d.h. auch bei erhöhter Zugabe von EFK 3000 ist keine Denaturierung des Albumins feststellbar. Die Standardsubstanz Natriumlaurylsulfat zeigt eine hohe eiweißschädigende Wirkung.

Akute orale Toxizität an Ratten

In bekannter Weise wurde der $LD_{50}$-Wert an Ratten bestimmt.

Der Wert für EFK 3000 lag über 46,4 ml/kg Körpergewicht bei oraler Aufnahme und liegt damit um ein Vielfaches höher als bei den meisten Tensiden, wo der Wert zwischen 1 und 2 ml/kg ist. Eine Berechnung der $LD_{50}$ war nicht möglich, da bei der höchsten vernünftigen Dosis von 46,4 ml/kg kein Tier starb.

Primäre Hautreizung am Kaninchen

Die primäre Hautreizung am Kaninchen dient zur Bestimmung der akuten Toxizität. Die Prüfung der Substanz in 32 %iger Wirkstoffkonzentration ergab entsprechend der nachfolgenden Tabelle keine Irritationseigenschaften; das als Vergleichsprodukt geprüfte EFK 500 hingegen zeigte noch eine leicht reizende Wirkung.

Tabelle 2

| Primäre Hautreizung am Kaninchen | |
| --- | --- |
| Methode: FDA § 1500.41<br>Akute Toxizität | |
| | 100 % Handelskonzentration |
| | (32 % WAS) |
| Haut-Reizindex<br>rasierte intakte Haut<br>rasierte scarifizierte Haut<br>Gesamtreiz-Index<br>Bewertung | EFK 3000<br>0<br>0,1<br>0,1<br>keine Irritationseigenschaften |
| Gesamtreiz-Index | Bewertung |
| 0,0-0,5<br>0,6-3,0<br>3,1-5,0<br>5,1-8,0 | nicht reizend<br>leicht reizend<br>mäßig reizend<br>stark reizend |

Augenreizung am Kaninchen nach Draize

Die Prüfung der Augenreizung am Kaninchen ergab für das erfindungsgemäße EFK 3000 auch in der hohen WAS-Konzentration von 33 % keinerlei Reizung, obwohl die Beurteilung nach Draize der Cornea, Iris und Conjunctiva bereits 5 Min. nach Applikation (siehe Tab. 3) erfolgte. Bei der Einstufung der Schleimhautverträglichkeit der meisten Tenside findet die erste Score-Beurteilung erst nach 24 h statt, in diesem Zeitraum können natürlich ein großer Teil der reversiblen Schädigungen (wie z.B. leichte Trübung, Schwellung, Sekretion) schon wieder abgeklungen sein. Die Draize-Untersuchung des niedrig molekularen EFK 500 ergab einen Gesamtreizindex von über 12,49, obwohl die erste Beurteilung erst nach 24 h erfolgte. Das Produkt EFK 3000 verhält sich wie die Kontrollsubstanz, die physiologische Kochsalzlösung.

Tabelle 3

| Augenreizung am Kaninchen | |
|---|---|
| (Draize-Test) Methode: FDA § 1500.42 | |
| | 100 % Handelskonzentration |
| | (32 % WAS) |
| Reizindex | EFK 3000 |
| 5 Minuten | 0 |
| 15 Minuten | 0 |
| 30 Minuten | 0 |
| 1 Stunde | 0 |
| 2 Stunden | 0 |
| 4 Stunden | 0 |
| 24 Stunden | 0 |
| 48 Stunden | 0 |
| 72 Stunden | 0 |
| Gesamtreizindex | 0 |

Die hochmolekularen Eiweißfettsäure-Kondensationsprodukte mit einer Molmasse zwischen 3000 und 7000 lassen sich in bekannter Weise zur Herstellung von kosmetischen, pharmazeutischen und technischen Reinigungsmitteln mit sehr guter Haut- und Schleimhautverträglichkeit verwenden. Sie können sowohl allein als auch in Kombination mit den bekannten anionischen, nichtionischen, kationischen und amphoteren Tensiden zur Herstellung dieser Präparate in den üblichen Mischungsverhältnissen eingesetzt werden.

Die folgenden Beispiele sollen die Einsatzmöglichkeiten der hochmolekularen Eiweißfettsäurekondensate erläutern, ohne sie jedoch darauf einzuschränken.

**Beispiel 1**

| Haarshampoo | |
|---|---|
| Eiweißfettsäurekondensat der mittleren Molmasse 3000, gemäß Beispiel 1 (EFK 3000) | 7,0 % |
| Alkyldiglykolethersulfatnatriumsalz (28 %ig) | 45,0 % |
| Parfümöl | 0,3 % |
| Natriumchlorid | 3,1 % |
| Konservierungsmittel, Parfümöl, Farbstoffe und Wasser | ad 100,0 % |

**Beispiel 2**

| Babyshampoo | |
|---|---|
| EFK 3000 | 28,0 % |
| Alkyldiglykolethersulfatnatriumsalz (28 %ig) | 7,0 % |
| Alkylamidopropylbetain | 5,0 % |

**Beispiel 3**

| Duschbad | |
| --- | --- |
| EFK 3000 | 5,0 % |
| Alkyltriglykolethersulfatnatriumsalz (28 %ig) | 39,0 % |
| Alkylamidobetain | 9,0 % |
| Natriumchlorid | 1,0 % |
| Wasser, Konservierungsmittel, Parfümöl | ad 100,0 % |

**Beispiel 4**

| Intimwaschmittel | |
| --- | --- |
| EFK 3000 | 8,0 % |
| Amidethersulfatnatriumsalz | 5,0 % |
| Alkylamidobetain | 3,0 % |
| Wasser, Wirkstoffe, Konservierungsmittel, Parfümöl | ad 100,0 % |

**Beispiel 5**

| Gesichtswaschmittel | |
| --- | --- |
| EFK 3000 | 4,0 % |
| Acylglutamat-Mononatriumsalz | 2,0 % |
| Lauroylsarkosidnatriumsalz | 4,0 % |
| Wasser, Parfümöl, Konservierungsmittel, Konsistenzgeber | ad 100,0 % |

**Beispiel 6**

| Handwaschmittel | |
| --- | --- |
| EFK 3000 | 2,0 % |
| Alkylsulfatnatriumsalz | 4,0 % |
| $\alpha$-Olefinsulfonat | 5,0 % |
| Sekundäres Alkansulfonatnatriumsalz | 2,0 % |
| Wasser, Konservierungsmittel, Parfümöl | ad 100,0 % |

**Beispiel 7**

8

| Handreinigungsmittel mit desinfizierender Wirkung | |
|---|---|
| EFK 3000 | 3,0 % |
| Sekundäres Alkansulfonatnatriumsalz | 10,0 % |
| Desinfizierende Wirksubstanz | 0,2 % |
| Wasser, Konservierungsmittel, Parfümöl | ad 100,0 % |

**Beispiel 8**

| Handgeschirrspülmittel | |
|---|---|
| EFK 3000 | 10,0 % |
| Sekundäres Alkansulfonatnatriumsalz (60 %ig) | 30,0 % |
| Alkyldiglykolethersulfatnatriumsalz (29 %ig) | 10,0 % |
| Wasser, Konservierungsmittel, Lösungsvermittler, Parfümöl | ad 100,0 % |

**Ansprüche**

1. Hochmolekulare Eiweißfettsäurekondensationsprodukte erhalten durch Umsetzung eines Mols eines Eiweißhydrolysats mit einer mittleren Molmasse von 3000 bis 7000 mit 0,5 bis 3 Mol eines $C_{12}$-$C_{18}$-Fettsäurechlorids in wäßrigem Medium bei einem pH-Wert von 7 bis 12.

2. Eiweißfettsäurekondensationsprodukte nach Anspruch 1, erhalten durch Umsetzung mit 2 bis 2,5 Mol eines $C_{12}$-$C_{18}$-Fettsäurechlorids.

3. Eiweißfettsäurekondensationsprodukte nach Anspruch 1 oder 2, wobei das Eiweißhydrolysat durch chemische oder enzymatische Hydrolyse von natürlichen Eiweißstoffen hergestellt ist.

4. Verwendung der Eiweißfettsäurekondensationsprodukte nach einem der Ansprüche 1 bis 3 in Wasch- und Reinigungsmitteln.

5. Wasch- und Reinigungsmittel, enthaltend das Eiweißfettsäurekondensationsprodukt nach einem der Ansprüche 1 bis 3.

Patentansprüche für folgenden Vertragsstaat: ES

1. Verfahren zur Herstellung von hochmolekularen Eiweißfettsäurekondensationsprodukten durch Umsetzung eines Mols eines Eiweißhydrolysats mit einer mittleren Molmasse von 3000 bis 7000 mit 0,5 bis 3 Mol eines $C_{12}$-$C_{18}$-Fettsäurechlorids in wäßrigem Medium bei einem pH-Wert von 7 bis 12.

2. Verfahren nach Anspruch 1, dadurch gekennzeichnet, daß das Eiweißhydrolysat mit 2 bis 2,5 Mol eines $C_{12}$-$C_{18}$-Fettsäurechlorids umgesetzt wird.

3. Verwendung der nach Anspruch 1 hergestellten Eiweißfettsäurekondensationsprodukte in Wasch- und Reinigungsmitteln.

EFK 3000          EFK 500

Fig. 1

Fig. 2

EP 0 417 619 A1

*Fig. 3*

## WASCHVERSUCHE
### MITTLERE SCHMUTZENTFERNUNG [%]

Fig. 4.

EP 0 417 619 A1

KONTROLLE
Na-BENZOAT

EFK 3000

EFK 500

Fig. 5

EP 0 417 619 A1

*Fig. 6*

# D 50

EP 0 417 619 A1

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | DD-A-2 676 62   (AKADEMIE DER WISSENSCHAFTEN DER DDR) <br> * Titelseite (Einleitung); Seite 1, Patentansprüche; Seite 2, Beispiel 1 * | 1-5 | C 11 D 1/32 |
| Y | DD-A-2 732 76   (AKADEMIE DER WISSENSCHAFTEN DER DDR) <br> * Seite 1, Patentansprüche und Charakteristik * | 1-5 | |
| Y | EP-A-0 203 418   (HENKEL KOMMANDITGESELLSCHAFT) <br> * Seite 2, Spalte 1, Zeile 5 - Spalte 2, Zeile 26; Seite 4, Spalte 6, Zeilen 5-41 * | 1-5 | |
| A | AMERICAN COSMETIC AND PERFUMERY, Band 87, März 1972, Seiten 65-67, Wheaton, IL, US; E.S. COOPERMAN: "Protein hydrolysates as skin moisturizers" <br> * Seite 65, Spalte 1, (unten); Seite 66, Spalte 2, (Diskussion) * | 1-5 | |
| A | PATENT ABSTRACTS OF JAPAN, Band 10, Nr. 335 (C-384)[2391], 13. November 1986; <br> & JP-A-61 140 515 (LION CORP.) 27-06-1986 <br> * Zusammenfassung Nr. 61-140515 * | 1-5 | RECHERCHIERTE SACHGEBIETE (Int. Cl.5) <br><br> C 11 D |

Der vorliegende Recherchenbericht wurde für alle Patentansprüche erstellt

| Recherchenort | Abschlußdatum der Recherche | Prüfer |
|---|---|---|
| Den Haag | 23 November 90 | KORSNER S.E. |